# EUROPÄISCHE PATENTANMELDUNG

(11) **EP 4 789 614 A1**
(43) Veröffentlichungstag der Anmeldung: **12.08.2026**
(21) Anmeldenummer: 25156167.6
(22) Anmeldetag: 06.02.2025
(51) Int. Cl.: A61B 6/04, A61B 6/50

(54) **KOMPRESSIONSEINHEIT ZUR KONTROLLE DER BRUSTKOMPRESSION BEI MAMMOGRAPHIEAUFNAHMEN**

(71) Anmelder: Siemens Healthineers AG, 91301 Forchheim (DE)
(72) Erfinder: Beister, Marcel, 91058 Erlangen (DE); Radicke, Marcus, 90587 Veitsbronn (DE); Syben, Christopher, 90556 Cadolzburg (DE)
(74) Vertreter: Siemens Healthineers Patent Attorneys

(57) **Zusammenfassung**

Eine Kompressionseinheit (100) zum Kontrollieren einer Kompression einer Brust (O) beim Erfassen einer Mammographieaufnahme umfasst eine erste Kompressionsplatte (106), eine zweite Kompressionsplatte und wenigstens einen optischen Sensor (102). Während dem Komprimieren wird die Brust (O) zwischen der ersten und der zweiten Kompressionsplatte komprimiert. Die erste Kompressionsplatte (106) ist dazu ausgebildet, während dem Komprimieren eine erste Kontaktfläche mit der Brust (O) auszubilden. Die erste Kompressionsplatte (106) umfasst wenigstens einen ersten Spiegel (109.1). Der wenigstens eine erste Spiegel (109.1) und der wenigstens eine optische Sensor (102) sind derart angeordnet, dass eine Spiegelung von wenigstens einem Teil der ersten Kontaktfläche von dem optischen Sensor (102) erfassbar ist.

## Beschreibung

Die vorliegende Offenbarung betrifft eine Kompressionseinheit für Mammographiesysteme, und insbesondere eine Kompressionseinheit, ein Mammographiesystem und ein Verfahren zur Kontrolle der Brustkompression bei Mammographieaufnahmen unter Verwendung von Spiegeln und optischen Sensoren.

Die vorliegende Erfindung betrifft das Gebiet der medizinischen Bildgebung, insbesondere die Mammographie zur Brustkrebsfrüherkennung. Bei der Mammographie wird typischerweise eine Brust zwischen zwei Kompressionsplatten komprimiert und mit Röntgenstrahlen durchleuchtet, um hochauflösende Aufnahmen des Brustgewebes zu erstellen. Eine korrekte Positionierung und gleichmäßige Kompression der Brust sind entscheidend für die Bildqualität und diagnostische Aussagekraft der Aufnahmen.

Herkömmliche Mammographiesysteme bieten nur begrenzte Möglichkeiten, die Brustkompression während der Untersuchung zu überwachen und zu optimieren. Oft verlassen sich Anwender auf visuelle Inspektion und ihre Erfahrung, um die Positionierung und Kompression zu beurteilen. Dies kann zu Variationen in der Bildqualität und potenziell zu Fehldiagnosen führen. Zudem erschwert die begrenzte Sicht insbesondere auf eine Kontaktfläche zwischen der Brust und wenigstens einer der Kompressionsplatten eine präzise Beurteilung von Faltenbildung oder ungleichmäßiger Kompression.

Bisherige Ansätze zur Verbesserung der Kompressionskontrolle, wie der Einsatz zusätzlicher optischer Sensoren, sind oft mit erhöhter Komplexität und Kosten verbunden. Die Integration mehrerer Sensoren kann zudem die Handhabung des Systems erschweren und den Arbeitsablauf beeinträchtigen.

Das zu lösende objektive Problem besteht darin, eine Vorrichtung bereitzustellen, die eine verbesserte Kontrolle und Optimierung der Brustkompression bei Mammographieaufnahmen ermöglicht, ohne dabei die Komplexität des Systems wesentlich zu erhöhen oder den Untersuchungsablauf zu beeinträchtigen.

Dieses objektive Problem wird durch eine Kompressionseinheit, ein Mammographiesystem und ein Verfahren gemäß den unabhängigen Ansprüchen gelöst. Vorteilhafte Merkmale und Weiterentwicklungen sind in den abhängigen Ansprüchen und in der folgenden Beschreibung aufgeführt.

Unabhängig von der verwendeten grammatikalischen Form sind Personen männlichen, weiblichen oder anderen Geschlechts mit dem Begriff eingeschlossen.

Im Folgenden wird die erfindungsgemäße Lösung sowohl in Bezug auf die beanspruchten Systeme als auch in Bezug auf die beanspruchten Verfahren beschrieben. Merkmale, Vorteile oder alternative Ausführungsformen hierin können den jeweils anderen beanspruchten Gegenständen zugeordnet werden und umgekehrt. Mit anderen Worten können Ansprüche für die Systeme mit Merkmalen verbessert werden, die im Zusammenhang mit den Verfahren beschrieben oder beansprucht sind. In diesem Fall werden die funktionalen Merkmale des Verfahrens durch objektive Einheiten des Systems verkörpert.

Die Erfindung betrifft in einem ersten Aspekt eine Kompressionseinheit zum Kontrollieren einer Kompression einer Brust beim Erfassen einer Mammographieaufnahme. Die Kompressionseinheit umfasst eine erste Kompressionsplatte, eine zweite Kompressionsplatte und wenigstens einen optischen Sensor. Während dem Komprimieren wird die Brust zwischen der ersten und der zweiten Kompressionsplatte komprimiert. Dabei ist die erste Kompressionsplatte dazu ausgebildet, während dem Komprimieren eine erste Kontaktfläche mit der Brust auszubilden. Dabei umfasst die erste Kompressionsplatte wenigstens einen ersten Spiegel. Dabei sind der wenigstens eine erste Spiegel und der wenigstens eine optische Sensor derart angeordnet, dass eine Spiegelung von wenigstens einem Teil der ersten Kontaktfläche von dem optischen Sensor erfassbar ist.

Die Kompressionseinheit kann dazu dienen, eine kontrollierte und überwachte Kompression der Brust während einer Mammographieuntersuchung zu ermöglichen.

Eine Mammographieaufnahme kann eine Röntgenaufnahme der Brust sein, die zur Früherkennung von Brustkrebs oder anderen Brusterkrankungen verwendet wird.

Die erste Kompressionsplatte und die zweite Kompressionsplatte können flache, stabile Platten sein, die dazu ausgebildet sind, die Brust zwischen sich einzuklemmen und zu komprimieren. Die erste und die zweite Kompressionsplatte kann aus einem röntgendurchlässigen Material hergestellt sein, beispielsweise aus Kunststoff oder Kohlefaser, um die Röntgenstrahlung während der Aufnahme nicht zu beeinträchtigen.

Der wenigstens eine optische Sensor kann ein bildgebendes Gerät sein, das in der Lage ist, optische Bilder aufzunehmen. Der optische Sensor kann beispielsweise eine Digitalkamera, eine Videokamera, eine Time of Flight Kamera, ein LIDAR Sensor oder ein anderer lichtempfindlicher Sensor sein. Der optische Sensor kann dazu dienen, Bilder der komprimierten Brust aufzunehmen, um die Qualität der Kompression zu überwachen.

Die erste Kontaktfläche kann der Bereich der Brust sein, der direkt mit der ersten Kompressionsplatte in Berührung kommt, wenn diese komprimiert wird. Diese erste Kontaktfläche kann wichtige Informationen über die Qualität der Kompression liefern, wie etwa die Gleichmäßigkeit des Drucks oder das Vorhandensein von Falten in der Haut.

Der wenigstens eine erste Spiegel kann ein reflektierendes Element sein, das in die erste Kompressionsplatte integriert oder an ihr angebracht ist. Der erste Spiegel kann aus einem Material hergestellt sein, das sowohl optisches Licht reflektiert und/oder röntgendurchlässig ist, um die Mammographieaufnahme nicht zu beeinträchtigen. Mögliche Materialien für den ersten Spiegel können dünne Metallbeschichtungen auf einem röntgendurchlässigen Substrat oder spezielle dielektrische Spiegel sein.

Die Anordnung des ersten Spiegels und des optischen Sensors ermöglicht es, dass eine Spiegelung von wenigstens einem Teil der ersten Kontaktfläche von dem optischen Sensor erfasst werden kann. Dies bedeutet, dass der optische Sensor indirekt Bilder der ersten Kontaktfläche zwischen der Brust und der ersten Kompressionsplatte aufnehmen kann, indem er die Reflexion dieser ersten Kontaktfläche in dem wenigstens einen Spiegel erfasst. Diese Anordnung kann es ermöglichen, Bereiche der Brust zu beobachten, die sonst für den optischen Sensor nicht direkt sichtbar wären.

Durch diese Konfiguration kann die Kompressionseinheit eine verbesserte Überwachung und Kontrolle der Brustkompression während der Mammographieaufnahme ermöglichen. Die Fähigkeit, die erste Kontaktfläche zwischen der Brust und der ersten Kompressionsplatte zu beobachten, kann dazu beitragen, Probleme wie ungleichmäßige Kompression oder Hautfalten zu erkennen und zu korrigieren, was zu einer verbesserten Bildqualität und einer genaueren Diagnose führen kann.

In einer weiteren vorteilhaften Ausführungsform der Kompressionseinheit kann die zweite Kompressionsplatte dazu ausgebildet sein, während dem Komprimieren eine zweite Kontaktfläche mit der Brust auszubilden. Die zweite Kompressionsplatte kann wenigstens einen zweiten Spiegel umfassen. Der wenigstens eine zweite Spiegel und der wenigstens eine optische Sensor können derart angeordnet sein, dass eine Spiegelung von wenigstens einem Teil der zweiten Kontaktfläche von dem optischen Sensor erfassbar ist.

Diese Konfiguration ermöglicht eine umfassende Überwachung des Kompressionsprozesses, indem sowohl die erste als auch die zweite Kontaktfläche zwischen der Brust und der ersten und der zweiten Kompressionsplatte beobachtet werden können.

Der zweite Spiegel kann in ähnlicher Weise wie der erste Spiegel in die zweite Kompressionsplatte integriert sein oder an dieser angeordnet sein. Die Anordnung des zweiten Spiegels kann so gewählt sein, dass der optische Sensor eine optimale Sicht auf die gespiegelte zweite Kontaktfläche erhält.

Der zweite Spiegel kann analog zu dem ersten Spiegel ausgebildet sein.

Die Möglichkeit, die erste und die zweite Kontaktfläche gleichzeitig zu überwachen, kann mehrere Vorteile bieten. Es kann eine vollständigere Beurteilung der Brustpositionierung und -kompression ermöglicht werden. Potenzielle Probleme wie ungleichmäßige Kompression oder Faltenbildung können auf beiden Seiten der komprimierten Brust erkannt werden. Dies kann zu einer verbesserten Qualität der Mammographieaufnahmen und einem erhöhten Patientenkomfort führen.

Das Design der ersten und/oder zweiten Kompressionsplatte kann modifiziert werden, um die Spiegelflächen zu integrieren. Dies kann durch die Verwendung von speziell beschichteten Bereichen auf den Oberflächen der ersten und/oder zweiten Kompressionsplatte erreicht werden. Alternativ können separate Spiegelelemente in die erste und/oder zweite Kompressionsplatten eingebaut werden. Die Integration der Spiegel kann so gestaltet sein, dass sie die primäre Funktion der ersten und/oder zweiten Kompressionsplatte nicht beeinträchtigt.

Werden der erste und/oder der zweite Spiegel durch separate Spiegelelemente ausgebildet, können diese die gesamte Oberfläche der ersten bzw. zweiten Kompressionsplatte abdecken. Alternativ können die Spiegelelemente kleiner als die gesamte Oberfläche der ersten bzw. zweiten Kompressionsplatte sein. Insbesondere kann dann der erste und/oder der zweite Spiegel mehrere Spiegelelemente umfassen. Diese mehreren Spiegelelemente können dann insbesondere versetzt bzw. in einem Winkel bzw. fächerartig zueinander angeordnet sein.

In einigen Ausführungsformen kann eine der Kompressionsplatten, typischerweise die zweite Kompressionsplatte, als Abdeckung eines Röntgendetektors dienen. In diesem Fall kann der zweite Spiegel in die Detektorabdeckung integriert sein. Dies kann eine effiziente Nutzung des vorhandenen Raums im Mammographiesystem ermöglichen und gleichzeitig die zusätzliche Funktionalität der Kompressionsüberwachung bieten.

Die Verwendung von zwei Spiegeln, jeweils an der ersten und zweiten Kompressionsplatte, kann eine symmetrische Überwachung des Kompressionsprozesses ermöglichen. Dies kann besonders vorteilhaft sein, um sicherzustellen, dass die Brust gleichmäßig komprimiert wird und keine Bereiche übersehen werden.

In einer weiteren vorteilhaften Ausführungsform des Kompressionseinheit kann der wenigstens eine optische Sensor an der ersten oder der zweiten Kompressionsplatte angeordnet sein.

Diese Anordnung des optischen Sensors ermöglicht eine kompakte und effiziente Integration in die Kompressionseinheit.

Durch die Platzierung des optischen Sensors direkt an der ersten oder der zweiten Kompressionsplatte kann eine optimale Sichtlinie zu dem ersten und/oder zweiten Spiegel und somit zu der ersten und/oder zweiten Kontaktfläche zwischen der Brust und der ersten und/oder zweiten Kompressionsplatte gewährleistet werden. Dies kann zu einer verbesserten Bilderfassung und Qualität der Kontrollaufnahmen führen.

Die Anordnung des optischen Sensors an der ersten oder der zweiten Kompressionsplatte kann verschiedene Vorteile bieten. Zum einen kann dadurch der Platzbedarf für zusätzliche Sensorhalterungen reduziert werden, was zu einem kompakteren Gesamtdesign der Kompressionseinheit beitragen kann. Zum anderen kann diese Anordnung die Verkabelung und Signalübertragung vereinfachen, da der wenigstens eine optische Sensor näher an den anderen elektronischen Komponenten der Kompressionseinheit positioniert ist.

Je nach spezifischer Ausführungsform kann der optische Sensor entweder an der ersten oder der zweiten Kompressionsplatte angebracht sein. Die Wahl der Platzierung kann von verschiedenen Faktoren abhängen, wie beispielsweise der Geometrie der Kompressionseinheit, der gewünschten Bildperspektive oder der Zugänglichkeit für Wartung und Kalibrierung.

In einigen Ausführungsformen kann der optische Sensor in die Oberfläche der ersten oder zweiten Kompressionsplatte integriert sein, um eine möglichst glatte und unterbrechungsfreie Oberfläche zu gewährleisten. In anderen Ausführungsformen kann der optische Sensor an der Außenseite oder am Rand der ersten oder zweiten Kompressionsplatte angebracht sein, um eine einfachere Installation oder Austauschbarkeit zu ermöglichen.

Die Anordnung des optischen Sensors an einer der Kompressionsplatten kann auch die Möglichkeit bieten, mehrere Sensoren an verschiedenen Positionen der ersten und/oder zweiten Kompressionsplatte anzubringen. Dies kann eine umfassendere Abdeckung des Untersuchungsbereichs ermöglichen und die Erfassung von Kontrollaufnahmen aus verschiedenen Blickwinkeln erleichtern.

In einer weiteren vorteilhaften Ausführungsform der Kompressionseinheit kann die erste oder die zweite Kompressionsplatte eine Abdeckung eines Röntgendetektors sein.

Durch die Kombination der Funktionen der ersten oder zweiten Kompressionsplatte und einer Detektorabdeckung in einem einzigen Bauteil kann die Gesamtkonstruktion des Mammographiesystems vereinfacht werden. Dies kann zu einer kompakteren Bauweise führen, was den Platzbedarf des Systems reduzieren und möglicherweise die Handhabung für das medizinische Personal erleichtern kann.

Die Integration kann auch potenzielle Vorteile für die Bildqualität bieten. Da die erste oder die zweite Kompressionsplatte direkt als Abdeckung des Röntgendetektors dient, kann der Abstand zwischen der komprimierten Brust und dem Röntgendetektor minimiert werden. Ein geringerer Abstand kann zu einer verbesserten Bildschärfe führen, da Streueffekte reduziert werden können.

Zusätzlich kann diese Konfiguration möglicherweise den Patientenkomfort verbessern. Durch die Reduzierung der Anzahl der Komponenten, die mit der Brust in Kontakt kommen, kann das Verfahren für die Patientin möglicherweise weniger invasiv erscheinen.

Die als Detektorabdeckung fungierende erste oder zweite Kompressionsplatte kann aus einem Material gefertigt sein, das sowohl für die Kompression der Brust geeignet ist als auch eine optimale Durchlässigkeit für Röntgenstrahlen aufweist. Mögliche Materialien können spezielle Kunststoffe oder Verbundwerkstoffe umfassen, die eine ausreichende mechanische Stabilität für die Kompression bieten und gleichzeitig eine minimale Abschwächung der Röntgenstrahlung verursachen.

In dieser Konfiguration kann die erste oder die zweite Kompressionsplatte, die als Detektorabdeckung dient, auch mit dem in früheren Aspekten beschriebenen Spiegel ausgestattet sein. Dies ermöglicht die Beibehaltung der Funktionalität zur optischen Überwachung der Brustkompression, während gleichzeitig die Vorteile der integrierten Detektorabdeckung genutzt werden.

In einer weiteren vorteilhaften Ausführungsform der Kompressionseinheit kann der wenigstens eine optische Sensor eine optische zweidimensionale Kamera sein. Eine optische zweidimensionale Kamera kann dazu ausgebildet sein, zweidimensionale Bilder der gespiegelten Kontaktflächen zwischen der Brust und den Kompressionsplatten aufzunehmen. Diese Bilder können verwendet werden, um die Positionierung und Kompression der Brust zu beurteilen.

Der wenigstens eine optische Sensor kann alternativ eine optische dreidimensionale Kamera sein. Eine optische dreidimensionale Kamera kann zusätzlich zur zweidimensionalen Bilderfassung auch Tiefeninformationen der gespiegelten Kontaktflächen erfassen. Dies kann eine genauere Beurteilung der Brustform und -kompression ermöglichen.

In einer alternativen Ausführungsform kann der wenigstens eine optische Sensor ein Time-of-Flight Sensor sein. Ein Time-of-Flight Sensor kann die Laufzeit von ausgesandten Lichtsignalen messen, um präzise Abstandsinformationen zu erfassen. Dies kann besonders nützlich sein, um feine Unterschiede in der Brustoberfläche während der Kompression zu erkennen.

Der wenigstens eine optische Sensor kann alternativ als Time-of-Flight Sensor Array ausgeführt sein. Ein solches Array kann aus mehreren Time-of-Flight Sensoren bestehen, die zusammenarbeiten, um ein größeres Sichtfeld abzudecken und detailliertere dreidimensionale Informationen über die Brustoberfläche zu liefern.

In einer alternativen Ausführungsform kann der wenigstens eine optische Sensor ein LIDAR Sensor sein. LIDAR (Light Detection and Ranging) Sensoren können hochauflösende dreidimensionale Punktwolken der gespiegelten Kontaktflächen erzeugen. Diese detaillierten Daten können für eine präzise Analyse der Brustform und -kompression während der Mammographie verwendet werden.

Der wenigstens eine optische Sensor kann auch als Matrixsensor ausgeführt sein. Ein Matrixsensor kann aus einer Vielzahl von einzelnen Sensorelementen bestehen, die in einem Raster angeordnet sind. Dies kann eine großflächige und gleichzeitig detaillierte Erfassung der gespiegelten Kontaktflächen ermöglichen.

Alle diese Sensortypen können ein großes Sichtfeld haben, was es ermöglicht, einen möglichst großen Bereich der gespiegelten Kontaktflächen zwischen der Brust und den Kompressionsplatten zu erfassen. Dies kann dazu beitragen, die Anzahl der benötigten Sensoren zu reduzieren und gleichzeitig eine umfassende Überwachung der Brustkompression zu gewährleisten.

In einer weiteren vorteilhaften Ausführungsform der Kompressionseinheit kann die Kompressionseinheit mehr als einen optischen Sensor umfassen. Die mehreren optischen Sensoren können derart angeordnet sein, einen möglichst großen Bereich der ersten und/oder zweiten Kontaktfläche mittels der Spiegelung zu erfassen.

Die Verwendung mehrerer optischer Sensoren kann verschiedene Vorteile bieten. Durch eine strategische Anordnung der Sensoren kann eine umfassendere Abdeckung der Kontaktflächen zwischen der Brust und den Kompressionsplatten erreicht werden. Dies kann ermöglichen, dass ein größerer Bereich der Brustoberfläche während der Kompression überwacht wird.

Die optischen Sensoren können beispielsweise an verschiedenen Positionen entlang der Ränder der ersten und/oder zweiten Kompressionsplatte angebracht sein. Eine solche Anordnung kann es ermöglichen, die erste und/oder zweite Kontaktfläche aus unterschiedlichen Winkeln zu erfassen und somit eine vollständigere Sicht auf die Kompression der Brust zu erhalten.

Durch die Erfassung eines möglichst großen Bereichs der ersten und/oder zweiten Kontaktfläche mittels mehrerer Sensoren kann eine detailliertere Analyse der Brustkompression ermöglicht werden. Dies kann zu einer verbesserten Kontrolle des Kompressionsprozesses führen. Beispielsweise können Unregelmäßigkeiten in der Kompression, wie Faltenbildung oder ungleichmäßiger Druck, möglicherweise früher und genauer erkannt werden.

Die Anordnung der mehreren optischen Sensoren kann je nach spezifischem Design der Kompressionseinheit variieren. Die Sensoren können symmetrisch oder asymmetrisch platziert sein, um eine optimale Abdeckung zu erreichen. Die genaue Anzahl und Position der Sensoren kann basierend auf Faktoren wie der Größe der ersten und/oder zweiten Kompressionsplatte, der erwarteten Brustgrößen und der gewünschten Auflösung der Überwachung bestimmt werden.

Die Verwendung mehrerer Sensoren kann auch die Zuverlässigkeit des Systems erhöhen. Falls ein Sensor ausfallen oder durch Verschmutzung beeinträchtigt sein sollte, können die anderen Sensoren weiterhin Daten liefern und so eine kontinuierliche Überwachung gewährleisten.

Die optischen Sensoren können eine Kombination verschiedener oben genannter Sensortypen umfassen. Die Kombination von Sensortypen in der Kompressionseinheit kann eine verbesserte Kontrolle und Optimierung des Kompressionsprozesses während der Mammographie ermöglichen. Dies kann zu einer genaueren Positionierung der Brust, einer gleichmäßigeren Kompression und letztendlich zu einer verbesserten Bildqualität der Mammographieaufnahmen führen.

Durch die umfassende Erfassung der ersten und/oder zweiten Kontaktflächen mittels mehrerer optischer Sensoren kann möglicherweise eine präzisere und individuellere Anpassung der Kompression erreicht werden. Dies kann zu einer Verbesserung der Bildqualität bei gleichzeitiger Minimierung des Unbehagens für die Patientin führen.

In einer weiteren vorteilhaften Ausführungsform der Kompressionseinheit können der wenigstens eine erste Spiegel und/oder der wenigstens eine zweite Spiegel aus einem optisch transparenten Material sein.

Ein geeignetes Material für die optisch transparenten Spiegel kann beispielsweise ein spezielles Glas oder ein transparenter Kunststoff sein, der eine hohe optische Reflektivität aufweist.

Vorteilhafterweise weist der erste und/oder der zweite Spiegel gleichzeitig eine geringe Röntgenabsorption auf.

Mögliche Materialien umfassen unter anderem dünne Schichten aus Siliziumdioxid, Aluminiumoxid oder bestimmte Polymere mit speziellen Beschichtungen.

Die optische Transparenz des ersten und/oder zweiten Spiegels kann vorteilhaft sein, da sie eine visuelle Inspektion der Brust während der Kompression und Bildgebung durch die erste und/oder zweite Kompressionsplatte hindurch ermöglicht. Gleichzeitig können der erste und/oder der zweite Spiegel ihre Funktion erfüllen, indem sie die Kontaktflächen zwischen der Brust und der ersten und/oder zweiten Kompressionsplatte für den optischen Sensor sichtbar machen.

Durch die Verwendung von optisch transparenten Materialien für den ersten und/oder zweiten Spiegel kann eine Kompressionseinheit realisiert werden, die sowohl eine präzise optische Kontrolle der Brustkompression als auch eine hochwertige Röntgenbildgebung ermöglicht. Dies kann zu einer verbesserten Genauigkeit und Zuverlässigkeit der Mammographieuntersuchung beitragen.

In einer weiteren vorteilhaften Ausführungsform des Kompressionseinheit kann der wenigstens eine erste und/oder der wenigstens eine zweite Spiegel keine oder nur eine geringe Röntgenabsorption aufweisen.

Diese Eigenschaft ist besonders vorteilhaft, wenn die Spiegel im Strahlengang der Röntgenstrahlung positioniert sind. Eine geringe Röntgenabsorption der Spiegel kann gewährleisten, dass die Spiegel die Mammographieaufnahme nicht oder nur minimal beeinflussen.

Die geringe Röntgenabsorption kann durch die Verwendung geeigneter Materialien für die Spiegel erreicht werden. Beispielsweise können dünne, röntgentransparente Substrate mit einer reflektierenden Beschichtung verwendet werden. Die reflektierende Beschichtung kann so dünn aufgetragen werden, dass sie die Röntgenstrahlung nur minimal absorbiert.

Eine weitere Möglichkeit besteht in der Verwendung von dielektrischen Spiegeln, die aus mehreren Schichten transparenter Materialien mit alternierenden hohen und niedrigen Brechungsindizes bestehen. Diese Art von Spiegeln kann so konzipiert werden, dass sie für sichtbares Licht hochreflektierend sind, während sie für Röntgenstrahlung nahezu transparent bleiben.

Die Homogenität der Röntgenabsorption der Spiegel kann ebenfalls von Bedeutung sein. Eine homogene Absorption über die gesamte Fläche des Spiegels kann dazu beitragen, Artefakte oder Verzerrungen in der Mammographieaufnahme zu minimieren. Dies kann durch eine gleichmäßige Dicke und Zusammensetzung des Spiegelmaterials erreicht werden.

Die geringe Röntgenabsorption der Spiegelmaterialien kann dazu beitragen, dass die Bildqualität der Mammographieaufnahme nicht beeinträchtigt wird. Dies kann besonders wichtig sein, wenn der erste und/oder zweite Spiegel im Strahlengang der Röntgenquelle positioniert sind. Insbesondere kann auf diese Weise sichergestellt werden, dass der Patientin keine höhere Röntgendosis als notwendig appliziert wird.

In einer optionalen Ausführungsform kann der wenigstens eine erste und/oder der wenigstens eine zweite Spiegel aus einem optisch transparenten Material sein und nur eine geringe Röntgenabsorption aufweisen.

Durch die Kombination der Eigenschaften optischer Transparenz und geringer Röntgenabsorption kann der wenigstens eine erste und/oder der wenigstens eine zweite Spiegel möglicherweise mehrere Funktionen in der Kompressionseinheit erfüllen. Diese Spiegel können aus Materialien hergestellt werden, die sowohl für sichtbares Licht als auch für Röntgenstrahlung durchlässig sind, während sie gleichzeitig eine ausreichende Reflektivität für die optische Bildgebung aufweisen. Mögliche Materialien für solche Spiegel können dünne Schichten aus Berylliumoxid, Borkarbid oder spezielle Kohlenstoff-Nanoröhren-Verbundwerkstoffe umfassen. Diese Materialien können in einigen Fällen eine hohe mechanische Stabilität, geringe Dichte und exzellente optische Eigenschaften kombinieren. Zusätzlich können mehrschichtige Strukturen aus abwechselnden Lagen von Siliziumnitrid und Siliziumdioxid verwendet werden, die als Bragg-Spiegel fungieren und gleichzeitig eine geringe Röntgenabsorption aufweisen. In manchen Ausführungsformen können auch Diamant-ähnliche Kohlenstoffbeschichtungen auf einem röntgentransparenten Substrat zum Einsatz kommen, die eine hohe Härte und chemische Beständigkeit mit den gewünschten optischen und röntgenphysikalischen Eigenschaften verbinden.

Durch die Verwendung von Spiegeln mit geringer und homogener Röntgenabsorption kann eine optimale Balance zwischen der optischen Überwachung des Kompressionsvorgangs und der Qualität der Mammographieaufnahme erzielt werden. Dies kann zu einer verbesserten Genauigkeit und Zuverlässigkeit des Mammographieverfahrens beitragen.

Die Erfindung betrifft in einem zweiten Aspekt ein Mammographiesystem. Das Mammographiesystem umfasst eine Röntgenquelle, einen Röntgendetektor und eine Kompressionseinheit nach einem der vorhergehenden Aspekte.

Die Kompressionseinheit kann zwischen der Röntgenquelle und dem Röntgendetektor derart positioniert sein, dass durch von der Röntgenquelle ausgesandte Röntgenstrahlung eine mit der Kompressionseinheit komprimierte Brust auf den Röntgendetektor projiziert wird.

Die Röntgenquelle kann eine Vorrichtung sein, die Röntgenstrahlung erzeugt. Die Röntgenquelle kann beispielsweise eine Röntgenröhre umfassen. Die Röntgenröhre kann einen Elektronenstrahl erzeugen, der auf eine Anode trifft und dabei Röntgenstrahlung emittiert. Die Röntgenquelle kann verschiedene Einstellungen für die Strahlungsintensität und -energie ermöglichen, um optimale Bildgebungsbedingungen für unterschiedliche Brustdichten und -dicken zu gewährleisten.

Der Röntgendetektor kann eine Vorrichtung sein, die die durch die Brust transmittierte Röntgenstrahlung erfasst und in ein digitales Bild umwandelt. Der Röntgendetektor kann beispielsweise ein Flachdetektor sein, der aus einer Matrix von Detektorelementen besteht. Jedes Detektorelement kann die einfallende Röntgenstrahlung in ein elektrisches Signal umwandeln, das dann digitalisiert wird. Der Röntgendetektor kann eine hohe räumliche Auflösung und einen großen dynamischen Bereich aufweisen, um feine Details in der Bruststruktur abzubilden.

Die Kompressionseinheit kann, wie in den vorhergehenden Aspekten beschrieben, eine erste und eine zweite Kompressionsplatte umfassen, zwischen denen die Brust komprimiert wird. Die Kompressionseinheit kann zusätzlich wenigstens einen optischen optische Sensor und wenigstens einen ersten und/oder wenigstens einen zweiten Spiegel umfassen, die eine Kontrolle der Brustkompression ermöglichen.

Die Anordnung der Röntgenquelle, der Kompressionseinheit und des Röntgendetektors kann so gestaltet sein, dass eine optimale Bildgebung der komprimierten Brust ermöglicht wird. Die Röntgenquelle kann oberhalb der Kompressionseinheit positioniert sein, um die Röntgenstrahlung von oben durch die komprimierte Brust zu senden. Der Röntgendetektor kann unterhalb der Kompressionseinheit angeordnet sein, um die durch die Brust transmittierte Strahlung zu erfassen.

In einer optionalen Ausführungsform kann das Mammographiesystem für die Durchführung von Tomosynthese-Aufnahmen ausgelegt sein. Bei der Tomosynthese kann die Röntgenquelle in einem begrenzten Winkelbereich um die komprimierte Brust bewegt werden, während mehrere Projektionsaufnahmen aus verschiedenen Winkeln erfasst werden. Diese Projektionsaufnahmen können anschließend zu einem dreidimensionalen Bildvolumen rekonstruiert werden.

Die Kompressionseinheit kann in diesem Fall so gestaltet sein, dass sie die Brust während der gesamten Aufnahmesequenz stabil in Position hält. Die optischen Sensoren und Spiegel der Kompressionseinheit können dabei möglicherweise genutzt werden, um die Stabilität der Kompression während der Bewegung der Röntgenquelle zu überwachen. Dies kann dazu beitragen, Bewegungsartefakte in den rekonstruierten Tomosynthese-Bildern zu minimieren.

Der Röntgendetektor kann für die Tomosynthese-Bildgebung optimiert sein, um eine schnelle Auslesegeschwindigkeit und eine hohe Dynamik zu ermöglichen. In einigen Ausführungsformen kann ein großflächiger Flachdetektor verwendet werden, der das gesamte Bildfeld abdeckt und so die Erfassung aller Projektionsaufnahmen ohne mechanische Bewegung des Detektors ermöglicht.

Diese Konfiguration des Mammographiesystems kann mehrere Vorteile bieten. Zum einen kann durch die Integration der Kompressionseinheit mit optischen Sensoren und Spiegeln eine präzise Kontrolle der Brustkompression während der Bildgebung ermöglicht werden. Dies kann zu einer verbesserten Bildqualität und einer reduzierten Strahlenbelastung für die Patientin führen. Zum anderen kann die Anordnung eine effiziente Durchführung der Mammographie ermöglichen, da die Kompression und die Bildgebung in einem einzigen System integriert sind.

In einer weiteren vorteilhaften Ausführungsform des Mammographiesystems kann die erste oder die zweite Kompressionsplatte eine Abdeckung eines Röntgendetektors ausbilden.

Die Abdeckung des Röntgendetektors kann aus einem röntgendurchlässigen Material gefertigt sein, um eine möglichst geringe Beeinträchtigung der Bildqualität zu gewährleisten. Gleichzeitig kann diese Abdeckung robust genug sein, um als erste oder zweite Kompressionsplatte zu fungieren und den für die Kompression erforderlichen Druck auf die Brust auszuüben.

Die Materialauswahl für die integrierte Kompressionsplatte/Detektorabdeckung kann verschiedene Aspekte berücksichtigen. Es können beispielsweise Verbundmaterialien verwendet werden, die sowohl eine ausreichende mechanische Stabilität für die Kompression als auch eine hohe Röntgendurchlässigkeit aufweisen.

Durch die Kombination der Funktionen von erster oder zweiter Kompressionsplatte und Detektorabdeckung in einem einzigen Bauteil kann die Gesamtkonstruktion des Mammographiesystems vereinfacht werden. Dies kann zu einer kompakteren Bauweise führen, was den Platzbedarf des Systems reduzieren und möglicherweise die Handhabung für das medizinische Personal erleichtern kann.

Die integrierte Lösung kann auch dazu beitragen, den Abstand zwischen der komprimierten Brust und dem Röntgendetektor zu minimieren. Ein geringerer Abstand kann potenziell die Bildschärfe verbessern, da Streueffekte reduziert werden können.

Zusätzlich kann diese Konfiguration die Möglichkeit bieten, Sensoren oder andere Komponenten direkt in die Kompressionsplatte/Detektorabdeckung zu integrieren. Beispielsweise können optische Sensoren oder Spiegel für die Überwachung der Brustkompression in dieses Bauteil eingebettet werden, ohne zusätzlichen Platz zu beanspruchen.

Die Integration kann auch aus hygienischer Sicht vorteilhaft sein, da weniger separate Oberflächen zu reinigen und zu desinfizieren sind. Dies kann die Wartung und Pflege des Systems vereinfachen.

Für die Patientin kann diese integrierte Lösung möglicherweise zu einem komfortableren Untersuchungserlebnis führen, da die Anzahl der Komponenten, die mit der Brust in Kontakt kommen, reduziert wird.

Die Erfindung betrifft in einem dritten Aspekt ein Verfahren zum Kontrollieren einer Kompression einer Brust beim Erfassen einer Mammographieaufnahme mit einem Mammographiesystem. Das Verfahren umfasst folgende Schritte:
- Positionieren der Brust zwischen der ersten und der zweiten Kompressionsplatte,
- Komprimieren der Brust zwischen der ersten und der zweiten Kompressionsplatte,
- Erfassen einer Kontrollaufnahme mit dem wenigstens einen optischen Sensor,
- Bereitstellen der Kontrollaufnahme,
- Kontrollieren der Kompression der Brust basierend auf der Kontrollaufnahme.

Das Positionieren der Brust zwischen der ersten und der zweiten Kompressionsplatte kann manuell durch medizinisches Fachpersonal oder automatisiert durch eine Positionierungseinheit des Mammographiesystems erfolgen. Die Brust kann dabei so ausgerichtet werden, dass eine optimale Abbildung des Brustgewebes in der späteren Mammographieaufnahme gewährleistet ist.

Das Komprimieren der Brust zwischen der ersten und der zweiten Kompressionsplatte kann durch eine Kompressionseinheit des Mammographiesystems durchgeführt werden. Die Kompression kann schrittweise erfolgen, um eine gleichmäßige Verteilung des Brustgewebes zu erreichen und Faltenbildung zu vermeiden.

Das Erfassen einer Kontrollaufnahme mit dem wenigstens einen optischen Sensor kann während oder unmittelbar nach dem Komprimieren erfolgen. Die Kontrollaufnahme kann eine Spiegelung wenigstens eines Teils der ersten Kontaktfläche an dem wenigstens einen ersten Spiegel und/oder eine Spiegelung wenigstens eines Teils der zweiten Kontaktfläche an dem wenigstens einen zweiten Spiegel abbilden. Der optische Sensor kann beispielsweise eine Kamera, ein Time-of-Flight Sensor oder ein LIDAR Sensor sein.

Das Bereitstellen der Kontrollaufnahme kann das Übertragen der Aufnahme an eine Auswerteeinheit des Mammographiesystems umfassen. Die Kontrollaufnahme kann in einem geeigneten Bildformat gespeichert und für die weitere Verarbeitung aufbereitet werden.

Das Kontrollieren der Kompression der Brust basierend auf der Kontrollaufnahme kann durch eine Auswerteeinheit des Mammographiesystems erfolgen. Dabei kann die Kontrollaufnahme auf Merkmale wie gleichmäßige Kompression, Abwesenheit von Falten oder optimale Positionierung der Brust analysiert werden. Die Auswertung kann automatisiert durch Bildverarbeitungsalgorithmen oder manuell durch medizinisches Fachpersonal erfolgen.

Die einzelnen Verfahrensschritte können in einer Schleife wiederholt werden, bis eine optimale Kompression und Positionierung der Brust erreicht ist. Bei einem negativen Ergebnis der Kontrolle kann eine Korrektur der Positionierung und/oder der Kompression der Brust erfolgen, gefolgt von einer erneuten Erfassung und Kontrolle. Bei einem positiven Ergebnis kann das Verfahren mit der Erfassung der eigentlichen Mammographieaufnahme fortgesetzt werden.

Das beschriebene Verfahren kann eine verbesserte Qualität der Mammographieaufnahmen ermöglichen, indem es eine optimale Kompression und Positionierung der Brust vor der eigentlichen Bildaufnahme sicherstellt. Dies kann zu einer erhöhten diagnostischen Genauigkeit und einer Reduzierung von Wiederholungsaufnahmen führen.

In einer weiteren vorteilhaften Ausführungsform des Verfahrens kann bei einem negativen Ergebnis der Kontrolle ein Korrigieren der Positionierung und/oder der Kompression der Brust erfolgen. Anschließend können die Schritte Erfassen der Kontrollaufnahme, Bereitstellen der Kontrollaufnahme und Kontrollieren der Kompression wiederholt werden.

Das Korrigieren der Positionierung kann beispielsweise eine Neupositionierung der Brust zwischen der ersten und der zweiten Kompressionsplatte umfassen. Hierbei kann die Brust so ausgerichtet werden, dass möglichst das gesamte Brustgewebe erfasst wird und Faltenbildung vermieden wird. Das Korrigieren der Kompression kann eine Anpassung des Kompressionsdrucks beinhalten. Der Kompressionsdruck kann erhöht werden, um eine bessere Gewebeauflösung zu erreichen, oder verringert werden, um den Patientenkomfort zu verbessern. Alternativ oder zusätzlich kann beim Korrigieren der Kompression die Brust erneut komprimiert werden, wobei darauf geachtet wird, dass sich keine Falten bilden.

Nach der Korrektur wird eine neue Kontrollaufnahme erfasst. Diese neue Kontrollaufnahme kann die Auswirkungen der Korrekturmaßnahmen zeigen und ermöglicht eine erneute Beurteilung der Brustkompression und -positionierung. Die Bereitstellung und Kontrolle der neuen Kontrollaufnahme erfolgt analog zu den vorherigen Schritten.

Dieser iterative Prozess des Korrigierens und erneuten Kontrollierens kann so lange wiederholt werden, bis ein zufriedenstellendes Ergebnis erreicht wird. Durch diesen Feedback-Loop kann die Qualität der Mammographieaufnahme verbessert und gleichzeitig der Patientenkomfort optimiert werden.

Bei einem positiven Ergebnis der Kontrolle kann ein Bereitstellen des positiven Ergebnisses erfolgen. Das positive Ergebnis kann anzeigen, dass die Brust optimal positioniert und komprimiert ist, um eine qualitativ hochwertige Mammographieaufnahme zu ermöglichen. Das Bereitstellen des positiven Ergebnisses kann beispielsweise durch ein visuelles oder akustisches Signal an den Bedienenden des Mammographiesystems erfolgen.

Das Bereitstellen des positiven Ergebnisses kann als Indikator dienen, dass die Vorbereitungen für die eigentliche Mammographieaufnahme abgeschlossen sind und mit der Röntgenbildgebung fortgefahren werden kann. Somit kann sichergestellt werden, dass die Mammographieaufnahme unter optimalen Bedingungen hinsichtlich Brustkompression und -positionierung durchgeführt wird.

In einer weiteren vorteilhaften Ausführungsform des Verfahrens kann der Verfahrensschritt des Bereitstellens des positiven Ergebnisses folgenden Verfahrensschritt initiieren: Erfassen einer Mammographieaufnahme der Brust.

Das Bereitstellen des positiven Ergebnisses kann als Auslöser für den Start des eigentlichen Mammographie-Bildgebungsprozesses dienen. Dieses positive Ergebnis kann anzeigen, dass die Brust korrekt positioniert und komprimiert ist, basierend auf der vorherigen Kontrolle der Kompression mittels der Kontrollaufnahme.

Das Initiieren des Erfassens einer Mammographieaufnahme kann verschiedene Schritte umfassen. Beispielsweise kann ein Signal an die Röntgenquelle gesendet werden, um die Emission von Röntgenstrahlung zu starten. Gleichzeitig kann der Röntgendetektor aktiviert werden, um die durch die Brust transmittierte Strahlung zu erfassen.

Der Übergang von der Kompressionskontrolle zur tatsächlichen Mammographieaufnahme kann automatisch erfolgen, sobald das System das positive Ergebnis registriert. Alternativ kann ein manueller Bestätigungsschritt durch medizinisches Fachpersonal erforderlich sein, bevor die Mammographieaufnahme initiiert wird.

Durch diese Vorgehensweise kann sichergestellt werden, dass die Mammographieaufnahme erst dann erfolgt, wenn die optimale Positionierung und Kompression der Brust erreicht ist. Dies kann zu einer verbesserten Bildqualität führen und die Notwendigkeit von Wiederholungsaufnahmen reduzieren. Darüber hinaus kann diese Methode dazu beitragen, die Strahlenbelastung für die Patientin zu minimieren, da die Wahrscheinlichkeit für technisch unzureichende Aufnahmen verringert wird.

Das Erfassen der Mammographieaufnahme kann verschiedene Bildgebungsparameter berücksichtigen, die basierend auf den Ergebnissen der Kompressionskontrolle optimiert wurden. Beispielsweise können die Belichtungszeit, die Strahlendosis oder der Aufnahmewinkel angepasst werden, um die bestmögliche diagnostische Bildqualität zu erzielen.

Die Erfindung betrifft in einem vierten Aspekt die Verwendung einer oben beschriebenen Kompressionseinheit in einem Mammographiesystem.

Die Kompressionseinheit kann in vorteilhafter Weise in einem Mammographiesystem eingesetzt werden, um die Kompression der Brust während der Mammographieuntersuchung zu kontrollieren und zu optimieren. Durch die Integration der Kompressionseinheit mit ihren optischen Überwachungsmöglichkeiten kann die Gesamtleistung und Zuverlässigkeit von Mammographieuntersuchungen verbessert werden.

Die Kompressionseinheit kann zwischen einer Röntgenquelle und einem Röntgendetektor des Mammographiesystems positioniert werden. In dieser Anordnung kann die Kompressionseinheit die Brust komprimieren, während gleichzeitig die Röntgenstrahlung von der Röntgenquelle durch die komprimierte Brust auf den Röntgendetektor projiziert wird.

Der wenigstens eine optische Sensor der Kompressionseinheit kann während und/oder nach dem Kompressionsvorgang wenigstens eine Kontrollaufnahme erfassen. Diese Kontrollaufnahmen können Spiegelungen der ersten und/oder zweiten Kontaktfläche zwischen der Brust und der ersten und/oder zweiten Kompressionsplatte abbilden. Basierend auf dieser wenigstens einen Kontrollaufnahme kann die Kompression der Brust überwacht und bei Bedarf angepasst werden.

Die Verwendung der Kompressionseinheit in einem Mammographiesystem kann mehrere Vorteile bieten. Die kontinuierliche optische Überwachung kann eine gleichmäßige und angemessene Kompression der Brust gewährleisten. Dies kann zu einer verbesserten Bildqualität der Mammographieaufnahmen führen, da eine optimale Kompression die Gewebedicke reduzieren und somit den Kontrast und die Detailerkennbarkeit in den Röntgenbildern erhöhen kann.

Darüber hinaus kann die Verwendung der Kompressionseinheit mit optischer Überwachung den Patientenkomfort verbessern. Durch die präzise Kontrolle der Kompression kann eine übermäßige oder ungleichmäßige Kompression vermieden werden, was das Unbehagen für die Patientin während der Untersuchung reduzieren kann.

Die Integration der Kompressionseinheit in das Mammographiesystem kann auch den Arbeitsablauf für das medizinische Personal optimieren. Die automatische Überwachung und Kontrolle der Brustpositionierung und -kompression kann die Notwendigkeit manueller Anpassungen reduzieren und somit die Effizienz der Untersuchung steigern.

Insgesamt kann die Verwendung der Kompressionseinheit in einem Mammographiesystem zu genaueren Diagnosen, verbessertem Patientenkomfort und effizienteren Arbeitsabläufen beitragen, was die Qualität und Zuverlässigkeit von Mammographieuntersuchungen insgesamt erhöhen kann.

Außerdem kann die Verwendung der Kompressionseinheit in einem Mammographiesystem dazu beitragen, eine applizierte Röntgendosis zu reduzieren, da fehlerhafte Aufnahmen vermieden werden können.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet.

Es zeigen:
FIG. 1 eine schematische Seitenansicht einer Kompressionseinheit für ein Mammographiesystem;
FIG. 2 eine schematische Draufsicht einer Kompressionseinheit für ein Mammographiesystem;
FIG. 3 eine schematische Seitenansicht einer Kompressionseinheit für ein Mammographiesystem;
FIG. 4 eine schematische perspektivische Ansicht eines Mammographiesystems; und
FIG. 5 ein Flussdiagramm eines Verfahrens zum Kontrollieren einer Kompression einer Brust beim Erfassen einer Mammographieaufnahme.

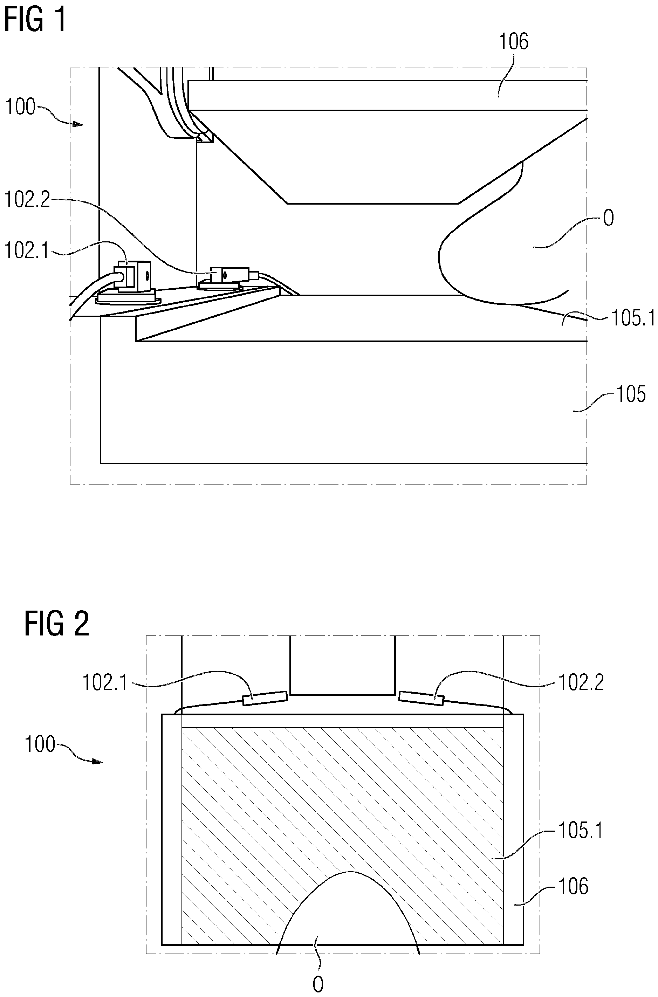

**FIG. 1** zeigt eine Seitenansicht einer Kompressionseinheit 100, bei der die Anordnung der Komponenten zur Brustuntersuchung und -kompression sichtbar ist. An der Oberseite kann eine insbesonders optisch und/oder für Röntgenstrahlung transparente erste Kompressionsplatte 106 angeordnet sein, welche dazu ausgebildet sein kann, die Brust O während der Bildgebung zu komprimieren. Unterhalb der ersten Kompressionsplatte 106 kann eine zweite Kompressionsplatte angeordnet sein. Die zweite Kompressionsplatte kann als Abdeckung 105.1 eines Röntgendetektors 105 ausgebildet sein, welche als Schutz für den darunter positionierten Röntgendetektor 105 dienen kann.

Die Kompressionseinheit 100 kann wenigstens einen optische Sensor 102.1, 102.2 zur Überwachung des Kompressionsvorgangs umfassen. Ein erster optischer Sensor 102.1 kann auf der linken Seite der Kompressionseinheit 100 angeordnet sein, um Bilder des Brustkompressionbereichs aufzunehmen. Ein zweiter optischer Sensor 102.2 kann ebenfalls von der Kompressionseinheit 100 umfasst sein.

Der Röntgendetektor 105 ist dazu ausgebildet sein, Röntgenbilder während einer Mammographieaufnahme zu erfassen.

Der Raum zwischen der ersten Kompressionsplatte 106 und der Abdeckung 105.1 des Röntgendetektors 105 kann der Bereich sein, in dem die Brust O während der Bildgebung positioniert werden kann. Die optisch transparente Beschaffenheit der ersten Kompressionsplatte 106 kann eine visuelle Inspektion der Brustpositionierung und -kompression ermöglichen.

Das Gesamtdesign der Kompressionseinheit 100 kann Kompressions-, Bildgebungs- und Überwachungsfähigkeiten integrieren, um genaue und kontrollierte Mammographieuntersuchungen zu ermöglichen.

Die Anordnung der Komponenten in der Kompressionseinheit 100 kann eine gleichzeitige Brustkompression, Röntgenbildgebung und optische Überwachung des Kompressionsvorgangs ermöglichen. Die erste Kompressionsplatte 106 und/oder die Abdeckung 105.1 des Röntgendetektors 105 können wenigstens einen ersten und/oder einen zweiten Spiegel umfassen. Der erste und/oder der zweite optische Sensor 102.1, 102.2 können wenigstens ein reflektiertes Bild (Kontrollaufnahme) einer ersten und/oder zweiten Kontaktfläche der Brust O mit der ersten Kompressionsplatte 106 und/oder der Abdeckung 105.1 des Röntgendetektors 105 aufnehmen.

**FIG. 2** zeigt eine schematische Darstellung einer vorteilhaften Ausführungsform einer Kompressionseinheit 100 für ein Mammographiesystem in einer Draufsicht. Die Kompressionseinheit 100 kann eine erste Kompressionsplatte 106 und eine zweite Kompressionsplatte umfassen. In der Draufsicht kann die erste Kompressionsplatte 106 als transparente Platte sichtbar sein, welche dazu ausgebildet sein kann, während dem Komprimieren eine erste Kontaktfläche mit einer Brust O auszubilden.

Unterhalb der ersten Kompressionsplatte 106 kann eine Abdeckung 105.1 eines Röntgendetektors 105 angeordnet sein. Diese Abdeckung 105.1 kann als zweite Kompressionsplatte dienen und dazu ausgebildet sein, während dem Komprimieren eine zweite Kontaktfläche mit der Brust O auszubilden.

Der Raum zwischen der ersten Kompressionsplatte 106 und der Abdeckung 105.1 des Röntgendetektors 105 kann den Bereich darstellen, in dem die Brust O während der Bildgebung positioniert werden kann.

Die Kompressionseinheit 100 kann wenigstens einen optischen Sensor 102.1, 102.2 umfassen. In einer vorteilhaften Ausführungsform können zwei optische Sensoren 102.1, 102.2 vorgesehen sein. Ein erster optischer Sensor 102.1 kann auf der linken Seite der Kompressionseinheit 100 angeordnet sein, während ein zweiter optischer Sensor 102.2 auf der rechten Seite positioniert sein kann. Diese Anordnung kann eine umfassende Überwachung des Kompressionsbereichs der Brust O ermöglichen.

Die erste Kompressionsplatte 106 kann wenigstens einen ersten Spiegel umfassen. In ähnlicher Weise kann die zweite Kompressionsplatte wenigstens einen zweiten Spiegel umfassen. Der wenigstens eine erste Spiegel und der wenigstens eine zweite Spiegel können derart angeordnet sein, dass eine Spiegelung von wenigstens einem Teil der ersten Kontaktfläche und/oder der zweiten Kontaktfläche von dem wenigstens einen ersten und/oder zweiten optischen Sensor 102.1, 102.2 erfassbar ist.

Die optisch transparente Natur der ersten Kompressionsplatte 106 kann eine visuelle Inspektion der Brustpositionierung und -kompression ermöglichen. Dies kann zu einer verbesserten Kontrolle und Anpassung des Kompressionsprozesses führen.

Das Gesamtdesign der Kompressionseinheit 100 kann Kompressions-, Bildgebungs- und Überwachungsfähigkeiten integrieren, um genaue und kontrollierte Mammographieuntersuchungen zu ermöglichen.

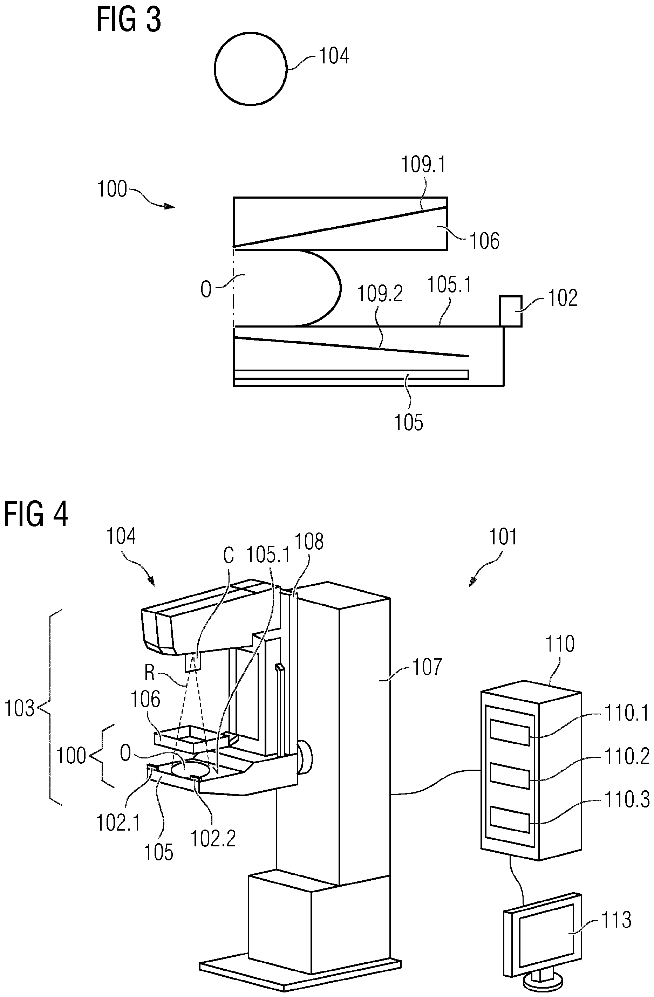

**FIG. 3** zeigt eine schematische Darstellung einer vorteilhaften Ausführungsform einer Kompressionseinheit 100 zum Kontrollieren einer Kompression einer Brust O beim Erfassen einer Mammographieaufnahme.

Die Kompressionseinheit 100 kann eine erste Kompressionsplatte 106 und eine zweite Kompressionsplatte umfassen. Die erste Kompressionsplatte 106 kann als obere Kompressionsplatte ausgebildet sein und die zweite Kompressionsplatte kann als untere Kompressionsplatte ausgebildet sein. Zwischen der ersten Kompressionsplatte 106 und der zweiten Kompressionsplatte kann eine Brust O positioniert und komprimiert werden.

Die zweite Kompressionsplatte kann als Abdeckung 105.1 eines Röntgendetektors 105 ausgebildet sein. Die als Abdeckung 105.1 des Röntgendetektors 105 fungierende Kompressionsplatte kann aus einem Material gefertigt sein, das sowohl für die Kompression der Brust O geeignet ist als auch eine optimale Durchlässigkeit für Röntgenstrahlen aufweist.

Die erste Kompressionsplatte 106 kann einen ersten Spiegel 109.1 umfassen. Der erste Spiegel 109.1 kann in die erste Kompressionsplatte 106 integriert sein. Die zweite Kompressionsplatte kann einen zweiten Spiegel 109.2 umfassen. Der zweite Spiegel 109.2 kann in die zweite Kompressionsplatte integriert sein.

Die Kompressionseinheit 100 kann weiterhin wenigstens einen optischen Sensor 102 umfassen. Der optische Sensor 102 kann an der zweiten Kompressionsplatte angeordnet sein. Der optische Sensor 102 kann beispielsweise eine optische Kamera, ein Time-of-Flight Sensor oder ein LIDAR Sensor sein.

Oberhalb der ersten Kompressionsplatte 106 kann eine Röntgenquelle 104 angeordnet sein. Die Röntgenquelle 104 kann dazu ausgebildet sein, Röntgenstrahlung R in Richtung der komprimierten Brust O auszusenden. Unterhalb der zweiten Kompressionsplatte kann ein Röntgendetektor 105 angeordnet sein. Der Röntgendetektor 105 kann dazu ausgebildet sein, die durch die komprimierte Brust O transmittierte Röntgenstrahlung R zu detektieren.

Der erste Spiegel 109.1 in der ersten Kompressionsplatte 106 und der zweite Spiegel 109.2 in der zweiten Kompressionsplatte können derart angeordnet sein, dass Spiegelungen von Teilen der ersten und/oder zweiten Kontaktfläche zwischen der komprimierten Brust O und der ersten bzw. zweiten Kompressionsplatte 106 von dem optischen Sensor 102 erfasst werden können. Der erste Spiegel 109.1 und der zweite Spiegel 109.2 können aus einem optisch transparenten Material gefertigt sein und eine geringe Röntgenabsorption aufweisen.

Durch die Anordnung der Spiegel 109.1, 109.2 und des optischen Sensors 102 kann eine visuelle Kontrolle der Kompression der Brust O ermöglicht werden. Der optische Sensor 102 kann Bilder der gespiegelten ersten und/oder zweiten Kontaktfläche erfassen. Diese Bilder können zur Beurteilung der Qualität der Kompression und zur Erkennung von möglichen Problemen wie Faltenbildung oder ungleichmäßiger Kompression verwendet werden.

**FIG. 4** zeigt ein Mammographiesystem 101 umfassend eine zuvor beschriebene Kompressionseinheit 100.

Das Mammographiesystem 101 kann eine Quellen-Detektor-Anordnung 103 umfassen. Die Quellen-Detektor-Anordnung 103 kann eine Röntgenquelle 104 und einen Röntgendetektor 105 umfassen. Die Röntgenquelle 104 kann dazu ausgebildet sein, Röntgenstrahlung R zu erzeugen und in Richtung des Röntgendetektors 105 auszusenden. Der Röntgendetektor 105 kann dazu ausgebildet sein, die durch eine zu untersuchende Brust O transmittierte Röntgenstrahlung R zu detektieren und in elektrische Signale umzuwandeln.

Zwischen der Röntgenquelle 104 und dem Röntgendetektor 105 kann eine Kompressionseinheit 100 angeordnet sein. Die Kompressionseinheit 100 kann gemäß der Beschreibung zu den Figuren 1 bis 3 ausgebildet sein. Die Kompressionseinheit 100 kann eine erste Kompressionsplatte 106 und eine zweite Kompressionsplatte umfassen. Die erste Kompressionsplatte 106 und zweite Kompressionsplatte können dazu ausgebildet sein, eine Brust O während einer Mammographieuntersuchung zu komprimieren. Die Kompressionseinheit 100 kann weiterhin wenigstens einen optischen Sensor 102 umfassen. Der optische Sensor 102 kann dazu ausgebildet sein, Kontrollaufnahmen in Form von Spiegelungen an dem ersten und/oder zweiten Spiegel der komprimierten Brust O zu erfassen.

Das Mammographiesystem 101 kann eine Säule 107 umfassen, an der ein Dreharm 108 befestigt sein kann. Der Dreharm 108 kann die Quellen-Detektor-Anordnung 103 und die Kompressionseinheit 100 tragen. Durch die Befestigung an der Säule 107 kann eine Rotation der Quellen-Detektor-Anordnung 103 und der Kompressionseinheit 100 um eine vertikale Achse ermöglicht werden. Dies kann die Aufnahme von Mammographiebildern aus verschiedenen Winkeln erlauben.

Das Mammographiesystem 101 kann für die Durchführung von Tomosynthese-Aufnahmen ausgelegt sein. Bei der Tomosynthese kann der Dreharm 108 die Röntgenquelle 104 in einem begrenzten Winkelbereich um die komprimierte Brust O bewegen, während mehrere Projektionsaufnahmen aus verschiedenen Winkeln erfasst werden. Diese Projektionsaufnahmen können anschließend zu einem dreidimensionalen Bildvolumen rekonstruiert werden.

Der Dreharm 108 kann so konstruiert sein, dass er eine präzise und stabile Bewegung der Röntgenquelle 104 während der Tomosynthese-Aufnahmen ermöglicht. Die Bewegung des Dreharms 108 kann durch einen Antriebsmechanismus gesteuert werden, der eine gleichmäßige und wiederholbare Rotation gewährleistet.

Während der Tomosynthese-Aufnahmen kann die Kompressionseinheit 100 die Brust O in einer stabilen Position halten. Die optischen Sensoren 102.1 und 102.2 können möglicherweise genutzt werden, um die Stabilität der Kompression während der Bewegung der Röntgenquelle 104 zu überwachen. Dies kann dazu beitragen, Bewegungsartefakte in den rekonstruierten Tomosynthese-Bildern zu minimieren.

Der Röntgendetektor 105 kann für die Tomosynthese-Bildgebung optimiert sein, um eine schnelle Auslesegeschwindigkeit und eine hohe Dynamik zu ermöglichen. In einigen Ausführungsformen kann ein großflächiger Flachdetektor verwendet werden, der das gesamte Bildfeld abdeckt und so die Erfassung aller Projektionsaufnahmen ohne mechanische Bewegung des Detektors ermöglicht.

Die Steuerung der Tomosynthese-Aufnahmen, einschließlich der Bewegung des Dreharms 108, der Aktivierung der Röntgenquelle 104 und der Synchronisation mit dem Röntgendetektor 105, kann durch ein Steuergerät 110 koordiniert werden. Das Steuergerät 110 kann auch die Rekonstruktion der Tomosynthese-Daten zu einem dreidimensionalen Bildvolumen durchführen.

Das Mammographiesystem 101 kann ein Steuergerät 110 umfassen. Das Steuergerät 110 kann eine Schnittstelle 110.1, eine Recheneinheit 110.2 und eine Speichereinheit 110.3 umfassen. Die Schnittstelle 110.1 kann zur Kommunikation mit anderen Komponenten des Mammographiesystems 101 dienen. Die Recheneinheit 110.2 kann zur Verarbeitung von Daten und Steuerung des Mammographiesystems 101 ausgebildet sein. Die Speichereinheit 110.3 kann zur Speicherung von Daten, Bildern und Steuerungsprogrammen dienen.

Das Mammographiesystem 101 kann weiterhin ein Terminal 113 umfassen. Das Terminal 113 kann eine Benutzerschnittstelle bereitstellen, über die ein Bediener das Mammographiesystem 101 steuern und Untersuchungsergebnisse und/oder Kontrollaufnahme anzeigen kann.

Die verschiedenen Komponenten des Mammographiesystems 101 können zusammenwirken, um eine Mammographieuntersuchung mit integrierter Kompressionskontrolle durchzuführen. Die Kompressionseinheit 100 kann die Brust O komprimieren, während der optische Sensor 102 Kontrollaufnahmen erfasst. Diese Kontrollaufnahmen können von der Recheneinheit 110.2 des Steuergeräts 110 analysiert werden, um die Qualität der Kompression zu überprüfen. Bei zufriedenstellender Kompression kann die Röntgenquelle 104 aktiviert werden, um ein Mammographiebild zu erzeugen, das vom Röntgendetektor 105 erfasst wird. Die erfassten Daten können in der Speichereinheit 110.3 gespeichert und über das Terminal 113 angezeigt werden.

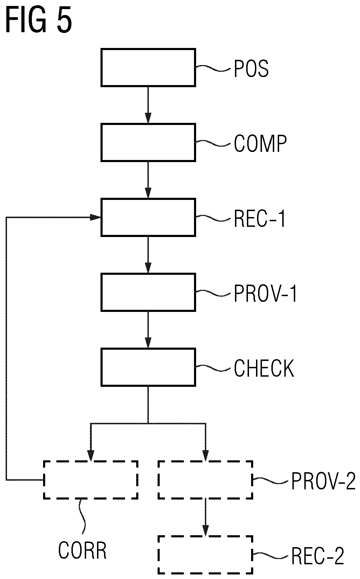

**FIG. 5** zeigt eine schematische Darstellung einer vorteilhaften Ausführungsform eines Verfahrens zum Kontrollieren einer Kompression einer Brust O beim Erfassen einer Mammographieaufnahme.

Gestrichelt dargestellte Verfahrensschritte sind optional.

Das Verfahren kann folgende Schritte umfassen:

Das Verfahren kann mit einem Positionieren POS der Brust O zwischen einer ersten und einer zweiten Kompressionsplatte 106 beginnen. Die Brust O kann dabei so platziert werden, dass eine optimale Bildgebung ermöglicht wird. Dieser Schritt kann manuell durch medizinisches Fachpersonal oder automatisiert durch eine Positionierungseinheit des Mammographiesystems 101 durchgeführt werden.

Nach dem Positionieren POS kann ein Komprimieren COMP der Brust O zwischen der ersten und der zweiten Kompressionsplatte 106 erfolgen. Die Kompression COMP kann dazu dienen, die Brustdicke zu reduzieren und das Brustgewebe zu spreizen, um eine verbesserte Bildqualität zu erzielen. Dieser Schritt kann durch eine Kompressionseinheit 100 (beispielsweise gemäß den Figuren 1 bis 3) des Mammographiesystems 101 ausgeführt werden, die schrittweise den Druck erhöht, um eine gleichmäßige Verteilung des Brustgewebes zu erreichen.

Im Anschluss an das Komprimieren COMP und/oder bereits während dem Komprimieren COMP kann ein Erfassen REC-1 einer Kontrollaufnahme mit wenigstens einem optischen Sensor 102 durchgeführt werden. Die Kontrollaufnahme kann eine Spiegelung wenigstens eines Teils einer ersten Kontaktfläche an wenigstens einem ersten Spiegel 109.1 und/oder eine Spiegelung wenigstens eines Teils einer zweiten Kontaktfläche an wenigstens einem zweiten Spiegel 109.2 abbilden. Diese Spiegelungen können es ermöglichen, die erste und/oder zweite Kontaktfläche zwischen der Brust O und der ersten und/oder zweiten Kompressionsplatte 106 zu visualisieren. Der optische Sensor 102 kann beispielsweise eine Kamera, ein Time-of-Flight Sensor oder ein LIDAR Sensor sein, der automatisch die Aufnahme erfasst.

Nach dem Erfassen REC-1 kann ein Bereitstellen PROV -1 der Kontrollaufnahme erfolgen. Die Kontrollaufnahme kann für eine weitere Analyse zur Verfügung gestellt werden. Dieser Schritt kann das Übertragen der Kontrollaufnahme an eine Auswerteeinheit des Mammographiesystems 101 umfassen, die die Daten in einem geeigneten Format speichert und aufbereitet. Alternativ oder zusätzlich kann das Bereitstellen PROV-1 ein Darstellen der Kontrollaufnahme an einem Monitor bzw. Bildschirm für ein medizinisches Fachpersonal und/oder einen Bediener des Mammographiesystems 101 umfassen.

Ein Kontrollieren CHECK der Kompression der Brust O kann basierend auf der Kontrollaufnahme durchgeführt werden. Dabei kann überprüft werden, ob die Brust O korrekt positioniert und komprimiert ist. Diese Auswertung kann automatisiert durch Bildverarbeitungsalgorithmen der Auswerteeinheit oder manuell durch medizinisches Fachpersonal erfolgen, die die Aufnahme auf Merkmale wie gleichmäßige Kompression oder Abwesenheit von Falten analysieren.

Bei einem negativen Ergebnis der Kontrolle CHECK kann ein Korrigieren CORR der Positionierung und/oder der Kompression der Brust O erfolgen. Dies kann eine Neupositionierung der Brust O und/oder eine Anpassung des Kompressionsdrucks und/oder der Kompression selbst durch die Kompressionseinheit 100 und/oder durch medizinisches Fachpersonal beinhalten. Anschließend können die Schritte des Erfassens REC-1 der Kontrollaufnahme, des Bereitstellens PROV-1 der Kontrollaufnahme und des Kontrollierens CHECK der Kompression wiederholt werden. Dieser iterative Prozess kann fortgesetzt werden, bis ein zufriedenstellendes Ergebnis erreicht ist.

Bei einem positiven Ergebnis des Kontrollierens CHCK kann ein Bereitstellen PROV-2 des positiven Ergebnisses erfolgen. Dies kann signalisieren, dass die Brust O optimal für die Mammographieaufnahme positioniert und komprimiert ist. Das Bereitstellen des positiven Ergebnisses kann beispielsweise durch ein visuelles oder akustisches Signal an den Bedienenden des Mammographiesystems erfolgen.

Das Bereitstellen PROV-2 des positiven Ergebnisses kann ein Erfassen REC-2 einer Mammographieaufnahme der Brust O initiieren. Die Mammographieaufnahme kann dann unter optimalen Bedingungen durchgeführt werden. Dafür kann ein Signal an die Röntgenquelle 104 gesendet werden, um die Emission von Röntgenstrahlung zu starten, während gleichzeitig der Röntgendetektor 105 aktiviert wird, um die transmittierte Strahlung zu erfassen.

Durch dieses Verfahren kann eine verbesserte Qualität und Konsistenz von Mammographieaufnahmen erreicht werden. Die Möglichkeit zur Korrektur CORR vor der eigentlichen Mammographieaufnahme kann dazu beitragen, Wiederholungsaufnahmen zu reduzieren und die Strahlenbelastung für die Patientin zu minimieren. Das Verfahren kann sowohl manuell durch geschultes medizinisches Personal als auch automatisiert durch die verschiedenen Komponenten der Kompressionseinheit 100 und/oder des Mammographiesystems 101 durchgeführt werden, wobei eine Kombination aus manuellen und automatisierten Schritten möglich ist.

Soweit nicht bereits ausdrücklich beschrieben, können einzelne Ausführungsformen oder deren einzelne Aspekte und Merkmale miteinander kombiniert oder ausgetauscht werden, ohne den Umfang der beschriebenen Erfindung zu begrenzen oder zu erweitern, sofern eine solche Kombination oder ein solcher Austausch sinnvoll und im Sinne dieser Erfindung ist. Vorteile, die in Bezug auf eine Ausführungsform der vorliegenden Erfindung beschrieben werden, sind, soweit anwendbar, auch vorteilhaft für andere Ausführungsformen der vorliegenden Erfindung.

## Patentansprüche

1. Kompressionseinheit (100) zum Kontrollieren einer Kompression einer Brust (O) beim Erfassen einer Mammographieaufnahme umfassend:
- eine erste Kompressionsplatte (106),
- eine zweite Kompressionsplatte und
- wenigstens einen optischen Sensor (102),
wobei während dem Komprimieren die Brust (O) zwischen der ersten und der zweiten Kompressionsplatte (106) komprimiert wird,
wobei die erste Kompressionsplatte (106) dazu ausgebildet ist, während dem Komprimieren eine erste Kontaktfläche mit der Brust (O) auszubilden,
wobei die erste Kompressionsplatte (106) wenigstens einen ersten Spiegel (109.1) umfasst, wobei der wenigstens eine erste Spiegel (109.1) und der wenigstens eine optische Sensor (102) derart angeordnet sind, dass eine Spiegelung von wenigstens einem Teil der ersten Kontaktfläche von dem optischen Sensor (102) erfassbar ist.

2. Kompressionseinheit (100) nach Anspruch 1,
wobei die zweite Kompressionsplatte dazu ausgebildet ist, während dem Komprimieren eine zweite Kontaktfläche mit der Brust (O) auszubilden,
wobei die zweite Kompressionsplatte wenigstens einen zweiten Spiegel (109.2) umfasst,
wobei der wenigstens eine zweite Spiegel (109.2) und der wenigstens eine optische Sensor (102) derart angeordnet sind, dass eine Spiegelung von wenigstens einem Teil der zweiten Kontaktfläche von dem optischen Sensor (102) erfassbar ist.

3. Kompressionseinheit (100) nach einem der vorhergehenden Ansprüche,
wobei der wenigstens eine optische Sensor (102) an der ersten oder der zweiten Kompressionsplatte (106) angeordnet ist.

4. Kompressionseinheit (100) nach einem der vorhergehenden Ansprüche,
wobei die erste oder die zweite Kompressionsplatte (106) eine Abdeckung eines Röntgendetektors (105) ist.

5. Kompressionseinheit (100) nach einem der vorhergehenden Ansprüche,
wobei der wenigstens eine optische Sensor (102) eine optische zweidimensionale Kamera oder eine optische dreidimensionale Kamera oder ein Time-of-Flight Sensor oder ein Time-of-Flight Sensor Array oder ein LIDAR Sensor ist.

6. Kompressionseinheit (100) nach einem der vorhergehenden Ansprüche,
umfassend mehr als einen optischen Sensor (102),
wobei die mehreren optischen Sensoren (102) derart angeordnet sind, einen möglichst großen Bereich der ersten und/oder zweiten Kontaktfläche mittels der Spiegelung zu erfassen.

7. Kompressionseinheit (100) nach einem der vorhergehenden Ansprüche,
wobei der wenigstens eine erste und/oder der wenigstens eine zweite Spiegel (109.1, 109.2) aus einem optisch transparenten Material sind.

8. Kompressionseinheit (100) nach einem der vorhergehenden Ansprüche,
wobei der wenigstens eine erste und/oder der wenigstens eine zweite Spiegel (109.1, 109.2) keine oder nur eine geringe Röntgenabsorption aufweisen.

9. Mammographiesystem (101) umfassend
- eine Röntgenquelle (104),
- einen Röntgendetektor (105),
- eine Kompressionseinheit (100) nach einem der Ansprüche 1 bis 8,
wobei die Kompressionseinheit (100) zwischen der Röntgenquelle (104) und dem Röntgendetektor (105) derart positioniert ist, dass durch von der Röntgenquelle (104) ausgesandte Röntgenstrahlung (R) eine mit der Kompressionseinheit (100) komprimierte Brust (O) auf den Röntgendetektor (105) projiziert wird.

10. Mammographiesystem (101) nach Anspruch 9,
wobei die erste oder die zweite Kompressionsplatte (106) eine Abdeckung des Röntgendetektors (105) ausbildet.

11. Verfahren zum Kontrollieren einer Kompression einer Brust (O) beim Erfassens einer Mammographieaufnahme mit einem Mammographiesystem (101) nach einem der Ansprüche 9 oder 10 umfassend folgende Schritte:
- Positionieren (POS) der Brust (O) zwischen der ersten und der zweiten Kompressionsplatte (106),
- Komprimieren (COMP) der Brust (O) zwischen der ersten und der zweiten Kompressionsplatte (106),
- Erfassen einer Kontrollaufnahme (REC-1) mit dem wenigstens einen optischen Sensor (102), wobei die Kontrollaufnahme (REC-1) die Spiegelung wenigstens eines Teils der ersten Kontaktfläche an dem wenigstens einen ersten Spiegels (109.1) und/oder die Spiegelung wenigstens eines Teils der zweiten Kontaktfläche an dem wenigstens einen zweiten Spiegels (109.2) abbildet,
- Bereitstellen der Kontrollaufnahme (PROV-1),
- Kontrollieren (CHECK) der Kompression der Brust (O) basierend auf der Kontrollaufnahme (REC-1).

12. Verfahren nach Anspruch 11,
- bei einem negativen Ergebnis der Kontrolle (CHECK):
- Korrigieren (CORR) der Positionierung und/oder der Kompression der Brust (O) und Wiederholen der Schritte Erfassen der Kontrollaufnahme (REC-1), Bereitstellen der Kontrollaufnahme (PROV-1) und Kontrollieren (CHECK) der Kompression,
- bei einem positiven Ergebnis:
- Bereitstellen des positiven Ergebnisses (PROV-2).

13. Verfahren nach Anspruch 12,
wobei der Verfahrensschritt des Bereitstellens des positiven Ergebnisses (PROV-2) folgenden Verfahrensschritt initiiert:
- Erfassen einer Mammographieaufnahme (REC-2) der Brust (O).

14. Verwendung einer Kompressionseinheit (100) nach einem der Ansprüche 1 bis 8 in einem Mammographiesystem (101).
